(19) Europäisches Patentamt
European Patent Office
Office européen des brevets

(11) EP 1 317 576 B1

# (12) EUROPEAN PATENT SPECIFICATION

(45) Date of publication and mention of the grant of the patent:
**19.04.2006 Bulletin 2006/16**

(21) Application number: **01964403.8**

(22) Date of filing: **23.08.2001**

(51) Int Cl.:
***D06M 13/46*** *(2006.01)* ***C08F 8/44*** *(2006.01)*
***C08G 63/688*** *(2006.01)* *D06P 3/52 (2006.01)*
*D06P 3/76 (2006.01)*

(86) International application number:
**PCT/US2001/026474**

(87) International publication number:
**WO 2002/022923 (21.03.2002 Gazette 2002/12)**

## (54) ANTIMICROBIAL POLYMERS

ANTIMIKROBIELLE POLYMERE

POLYMERES ANTIMICROBIENS

(84) Designated Contracting States:
**AT BE CH CY DE DK ES FI FR GB GR IE IT LI LU MC NL PT SE TR**

(30) Priority: **15.09.2000 US 662999**

(43) Date of publication of application:
**11.06.2003 Bulletin 2003/24**

(73) Proprietor: **The Regents of the University of California**
**Oakland, California 94607-5200 (US)**

(72) Inventors:
- **SUN, Gang**
  **Davis, CA 95616 (US)**
- **KIM, Young, Hee**
  **Berkeley, CA 94720-1620 (US)**

(74) Representative: **Jones, Keith William et al**
**Murgitroyd & Company**
**Scotland House**
**165-169 Scotland Street**
**Glasgow G5 8PL (GB)**

(56) References cited:
**EP-A- 0 200 903**
**WO-A-00/15897**
**WO-A-01/14629**
**GB-A- 1 474 482**
**US-A- 3 643 270**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 317 576 B1

## Description

## FIELD OF THE INVENTION

In general, this invention relates to novel polymeric materials, and in particular, to antimicrobial polymer compositions.

## BACKGROUND OF THE INVENTION

Novel textile materials with particular functions that provide health benefits to people have recently attracted attention from researchers *(see,* Scott, E., *et al., J. Applied Bacteriology,* **68**:271-278 (1990); Yang, Y., *et aL, Textile Chemist and Colorist & American Dyestuff Reporter*, **32(4)**:44-49 (2000); Sun, K. P., *et al., Journal of Electrostatics,* **44**:125-129 (1998); Eckhardt C., *et al., Textile Chemist and Colorist & American Dyestuff Reporter,* **32(4)**:21-23 (2000)). Examples of the functional textiles include antimicrobial, anti-toxic chemical, anti-UV, and anti-radiation fabrics and clothing (*see,* Sun, K. P., *et al., Journal of Electrostatics,* **44**:125-129 (1998); Eckhardt C., *et al., Textile Chemist and Colorist & American Dyestuff Reporter,* **32(4)**:21-23 (2000); Ko, L. L., *et al., Textile Chemist and Colorist & American Dyestuff Reporter,* **32(2)**:34-38 (2000)). In order to bring these special properties to textile materials, functional chemical agents are incorporated into polymer molecules of the fibers. The incorporation of the chemicals into polymers relies on intermolecular interactions between fiber polymers and foreign chemicals, such as van der Waals, dipole-dipole, covalent and ionic interactions, as well as hydrogen bonding. For instance, in dyeing of textiles, dye molecules can either form covalent bonds or strong secondary interactions such as dipole-dipole, and hydrogen bonds with polymers. Reactive dyeing of cotton fabrics uses covalent bonds formed between dyes and cellulose to achieve excellent color washfastness. On the other hand, disperse dyeing of polyesters and many synthetic fibers employ strong dipole-dipole interactions with the polymers to provide the same color durability.

Moreover, silicone based polymeric quaternary ammonium salts have been incorporated into textiles for achieving durable antimicrobial properties due to enhanced interactions between the macromolecular salts and fibers *(see,* Isquith, A. J., *et al., Applied Microbiology,* **24**:859-863 (1972)). In addition, ionic bonds were utilized in antimicrobial treatment of nylon fabrics by utilizing acid dyes as bridges to interact with quaternary ammonium salts (*see,* Kim, Y. H., *et. al., Textile Research Journal,* in press).

In recent years, there has been the development of innovative technologies that prepare durable functional textiles, including biocidal and pesticide-protective clothing. For example, cellulose related materials have been generated by using covalent bonding (*see,* Sun, G., *et al., Textile Chemist and Colorist,* **30(6)**:26 (1998); Sun, G., *et al., Textile Chemist and Colorist,* **31(5)**:31 (1999)); Sun, G., *et al., Textile Chemist and Colorist,* **31(1)**:21-24 (1999)). However, due to the lack of reactive groups in most synthetic fibers, there exist limited practical options *i.e*. few intermolecular interactions to achieve durable functions on polymers.

PCT Publication WO 00/15897 published March 23, 2000, to Sun *et al.,* discloses durable and refreshable antimicrobial polymers such as textiles, that have excellent colorfastness and washfastness. The textiles are suitable for sportswear, antiodor carpets, films, plastics, toys and medical uses. In that invention, dye molecules are used as connectors or bridges between the textile and antimicrobial agents.

Despite the advances made by PCT Publication WO 00/15897, there exists a need for new antimicrobial polymers for use in fibers and textiles and methods of producing the same. The present invention provides these and other needs.

## SUMMARY OF THE INVENTION

By examining the structures of synthetic fibers, it has been surprisingly found that certain interactions such as ionic interactions, can be employed in the functional finishing of synthetic fibers. Reactive or functional groups in polymers (*e.g.* acrylics) such as anionic groups, can be employed as a point of attachment for antimicrobial agents. Dye molecules having a complementary functional group, such as a cationic group, can penetrate into and reside in the polymers to form strong interactions, such as ionic interactions, with their counterparts (*e.g.*, anionic group).

As such, in one embodiment, the present invention provides an antimicrobial polymer composition, comprising a polymer having at least one functional monomeric unit of Formula I. The functional monomeric unit serves as a point of attachment for interaction with the antimicrobial agent. In certain instances, the antimicrobial composition employs ionic interactions between polymers such as acrylic polymers, and antimicrobial agents such as quaternary ammonium salts. In certain aspects, the finishing conditions, polymer morphology, and structure of the antimicrobial agents play important roles in achieving durable performance of the compositions. Preferably, the composition comprises a synthetic organic polymer such as an acrylic polymer, or cationic dyeable polyester. The polymer can be a fiber woven into a textile.

Contamination of polymeric materials by microorganisms such as pathogenic bacteria, odor-generating bacteria, molds, fungi and viruses is of great concern in the medical industry, the food and restaurant industries, as well as in consumer products. Thus, the antimicrobial textiles of the present invention can be used in a wide variety of applications. Suitable applications include surgeon's gowns, caps, masks, surgical covers, patient drapes, carpeting, bedding material, underwear, socks, sportswear, and healthcare uniforms. In a preferred aspect, the application is sportswear and socks.

The antimicrobial polymer, comprises at least one functional monomeric unit of Formula I:

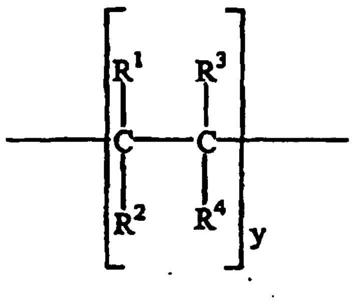

I

In Formula I, $R^1$, $R^2$ and $R^3$ are each independently, hydrogen, halogen, hydroxyl, cyano, $(C_1\text{-}C_6)$alkyl, $(C_{2\text{-}}C_6)$ alkenyl, $(C_1\text{-}C_6)$alkoxy, $(C_1\text{-}C_6)$alkylcarbonyl, $(C_1\text{-}C_6)$alkylcarboxyl, aldehydo, amido, aryl and heterocyclyl.

$R^4$ in Formula I, is $-CO_2^-X^+$, $SO_3^-X^+$, $-O^-X^+$, $-PO_4^{-2}ZX^+$ and $-PO_3^{-2}ZX^+$, wherein X is a quaternary ammonium salt selected from the group consisting of a cetylpyridinium ion, a benzyldimethylhexadecylammonium ion, a N-(3-chloro-2-hydroxypropyl)-N,N-dimethyldodecylammonium ion, a 1,3-Bis-(N,N-dimethyldodecylammonium ion)-2-propanol, a N-(1-(2,3-dioleoyloxy)propyl)-N,N,N-trimethylammonium ion, a N-(1-(2,3-dioleyloxy)propyl)-N,N,N-trimethylammonium ion, a N,N-dioleyl-N,N-dimethylammonium ion, a 1,2-dioleoyloxy-3-(N,N,N-trimethylamino)propane ion and mixtures thereof. For the phosphate ($-PO_4^{-2}$) group and the phosphonate ($-PO_3^{-2}$) group, the additional valence is be filled with Z, which is a hydrogen atom or an alkaline earth metal (*e.g.,* sodium, potassium, *etc.*).

The index "y" is an integer from 1 to 250 inclusive.

In a preferred embodiment, the antimicrobial polymer comprising the functional monomeric unit of Formula I is a long-chain synthetic acrylic polymer or fiber comprising at least 85% by weight of acrylonitrile units $(-CH_2\text{-}CH[CN]\text{-})_x$. Alternatively, the antimicrobial polymer is modacrylic polymer of fiber having less than 85% by weight of acrylonitrile units, but at least 35% by weight of acrylonitrile units.

In yet another embodiment, the present invention provides a method for making an antimicrobial polymer composition as claimed in any preceding claim, said method comprising:

finishing or dyeing a polymer in an aqueous solution comprising a quaternary ammonium salt, wherein said polymer has a functional monomeric unit with an antimicrobial agent to form a polymer having said antimicrobial agent attached thereto;

wherein said functional monomeric unit comprises a member selected from the group consisting of a carboxylate group, a sulfonate group, an alkoxide group, a phosphate group and a phosphonate group; and wherein said antimicrobial agent is a quaternary ammonium salt. The polymers thus produced possess biocidal properties that are effective in myriad medical and related healthcare products, sportswear and hygienic-use textiles.

Those and other embodiments and advantages will become more apparent when read with the accompanying figures and detailed description that follows.

## BRIEF DESCRIPTION OF THE DRAWINGS

**FIGS. 1 A-B** illustrate various embodiments of antimicrobial agents (A) quaternary ammonium salts and (B) basic dyes.

**FIG. 2** illustrates one embodiment of an effect of an antimicrobial agent uptake versus temperature increase.

**FIGS. 3 A-B** illustrate embodiments of effects of concentration of two quaternary ammonium salts (A) cetylpyridinium chloride (CPC) and (B) benzyldimethylhexadedyl ammonium chloride (BDHAC) at a temperature equal to 100°C, pH equal to 5, and $Na_2SO_4$ equal to 5%.

**FIGS. 4 A-B** illustrate embodiments of effect of pH on uptake of two quaternary ammonium salts solutions (A)

cetylpyridinium chloride (CPC) and (B) benzyldimethylhexadedyl ammonium chloride (BDHAC) at 4% concentration.

**FIG. 5** illustrates effect of durability of quaternary ammonium salt treated acrylic fabrics (treated by 4% owf, 100 °C for 90 minutes).

**FIG. 6** illustrates one embodiment of the add-ons of CPC on various fabrics after rinsing (Orlon(#864), Acrylan (#981), and cationic dyeable polyester (PET) (#763H)).

**FIG. 7** illustrates various add-on percent of the quaternary ammonium salts on the fabrics in Figure 6 after rinsing.

**FIG. 8** illustrates antimicrobial functions of acrylics finished by basic dyes (1% owf) or CPC (1%, owf). R9 is basic red 9; 014 is basic orange 14; B9 is basic blue 9; B69 is basic blue 69; and B22 is basic blue 22.

**FIG. 9** illustrates antimicrobial function of acrylics treated by a mixture of a basic dye and CPC.

## DETAILED DESCRIPTION OF THE INVENTION AND PREPARED EMBODIMENTS

I. The Composition

In embodiment the present invention provides an antimicrobial polymer composition comprising a polymer having at least one functional monomeric unit of Formula I. The terms "antimicrobial," "microbicidal," or "biocidal" as used herein, refer to the ability to kill at least some types of microorganisms, or to inhibit the growth or reproduction of at least some types of microorganisms. The polymers, fibers, films and textiles prepared in accordance with the present invention have microbicidal activity (antimicrobial) against a broad spectrum of microorganisms. For example, if the polymer is a textile, the textiles have microbicidal activity against representative gram-positive (such as *Staphylococcus aureus*) and gram-negative bacteria (such as *Escherichia coli*). Moreover, the microbicidal activity of such textiles is readily regancrable.

A wide variety of polymers can be used in the present invention. Suitable polymers include, bur are not limited to, fibers from plants, polymers from animals, natural organic polymers, synthetic organic polymers and inorganic substances. In a preferred aspect, synthetic organic polymers such as acrylic polymers are used. In certain aspects, the acrylic polymers suitable for use in the present invention have a number average molecular weight of about 40,000 to about 60,000 or about 1000 to about 1500 repeat units. The weight average molecular weight is about 90,000 to about 140,000, with the polydispersity index between about 1.5 to about 3.0.

Moreover, in certain embodiments, the polymer is a plurality of polymers. Suitable plurality of polymers include, but are not limited to, fibers, films, textiles and plastics. In preferred aspects, the antimicrobial fibers are acrylic fibers. As used herein, the term "acrylic fiber" means any manmade fiber derived from acrylic resins comprising a minimum of 85% acrylonitrile, contained therein. Acrylic fiber is a manufactured fiber in which the fiber forming substance is any long-chain synthetic polymer comprising at least 85% by weight of acrylonitrile units $(\text{-}CH_2\text{-}CH[CN]\text{-})_x$. As used herein the term "modacrylic fiber" means a fiber having less than 85% by weight of acrylonitrile units, but at least 35% by weight of acrylonitrile units.

In a preferred embodiment, the acrylic fibers used in the present invention are made from acrylonitrile and at least one other functional monomer. The functional, monomers have a functional group, preferably an ionic functional group. Selected from a carboxylate ($-CO_2$) group, a sulfonate ($SO_3^-$) group, a hydroxide (-OH) group, a phosphate ($-PO_4^{-2}$) group and a phosphonate ($-PO_3^{-2}$) group. In a preferred aspect, the functional monomer comprises a negatively charged functional monomer such as a sulfonate ($SO_3^-$) group. Functional monomers comprising a sulfonate group include, but are not limited to, sodium styrenesulfonate, sodium methyallyl sulfonate and sodium sulfophenyl methallyl ether.

Suitable acrylic fibers are produced by various manufactures. Suitable acrylic fibers for use in the present invention include, but arc not limited to, MicroSupreme®, Cresloft™, Creslan® Plus, BioFresh™, WeatherBloc™ (commercially available from Sterling Fibers, Inc.); Dralon™ (commercially available from Bayer Inc.) and Acrilan®, Bounce-Bac®, Duraspun®, Pil-Trol®, Sayelle®, Sno-Brite™, The Smart Yarns®, Wear-Dated® and Wintuk® (commercially available from Solutia Inc.). Other acrylic fibers include Orlon™, Acrilin® acrylic, Dolan®, Dralon®, Vinyon N®, Dynel®, Verel®, SEF modacrylic®. Those of skill in the art will know of other manufactures and trade names of acrylic fibers suitable for use in the present invention.

In another embodiment, the antimicrobial polymer of present invention is a textile. The textiles suitable for the present invention include, but are not limited to, naturally occurring fibers from plants, such as cellulose, cotton, linen, hemp, jute and ramie. They include polymers from animals, based upon proteins and include, but are not limited to, wool, mohair, vicuna and silk. Textiles also include manufactured fibers based upon natural organic polymers such as, rayon, lyocell, acetate, triacetate and azlon. Textiles suitable for use in the present invention include synthetic organic polymers which include, but are not limited to, acrylic, aramid, nylon, olefin, polyester, spandex, vinyon, vinyl and graphite. Textiles also include inorganic substances such as glass, metallic and ceramic.

The antimicrobial polymer compositions of the present invention comprise an antimicrobial agent attached to the functional monomeric unit. The antimicrobial agent can be attached to the functional monomeric unit via various

linkages including, but not limited to, covalent, ionic, hydrogen bonding, Van der Waals' forces, or mechanical bonding, *etc.* Preferably, the linkage is ionic. Wide ranges of antimicrobial agents exist. The antimicrobial agent is a quaternary ammonium salt. Fig. 1A illustrates suitable aromatic and aliphatic quaternary ammonium salts that are suitable antimicrobial agents. Suitable antimicrobial agents include, but are not limited to, cetylpyridinium chloride, benzyldimethylhexadecylammonium chloride, dodecyltrimethyl ammonium bromide, N-(3-chloro-2-hydroxypropyl)-N,N-dimethyldodecylammonium chloride, 1,3-Bis-(N,N-dimethyldodecylammonium chloride)-2-propanol, N-(1-(2,3-dioleoyloxy)propyl)-N,N,N trimethylammonium chloride, N-(1-(2,3-dioleyloxy)propyl)-N,N,N-trimethylammonium chloride, N,N-dioleyl-N,N-dimethylammonium chloride, 1,2-dioleoyloxy-3-(N,N,N-trimethylamino)propane chloride and mixtures thereof.

In certain aspects, the reactive sites on the functional monomers serve as a point of attachment to the complementary functional group on the antimicrobial agent. The functional group on the antimicrobial agent such as a cationic functional group, can penetrate into and reside in the fibers to form strong interactions, such as ionic interactions, with the counterpart of the polymers (*e.g.,* anionic sulfonate group). For example, a sulfonate ($SO_3^-$) group on the polymer, will become ionically bonded to the quaternary ammonium salt ($-NH_4^+$).

## II. The Antimicrobial Polymer

The antimicrobial polymer, comprises at least one functional monomeric unit of Formula I:

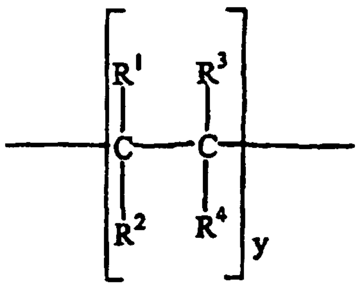

wherein $R^1$, $R^2$, $R^3$, $R^4$X, Z and y have been previously defined.

In a preferred embodiment, the antimicrobial polymer comprising the functional monomeric unit of Formula I is a long-chain synthetic acrylic polymer or fiber comprising at least 85% by weight of acrylonitrile units $(-CH_2-CH[CN]-)_x$, wherein x is about 1000 to about 1500. Alternatively, the antimicrobial polymer is modacrylic polymer of fiber having less than 85% by weight of acrylonitrile units, but at least 35% by weight of acrylonitrile units.

In certain preferred aspects, the acrylic polymers having the monomeric unit of formula I have a number average molecular weight of about 40,000 to about 60,000 or about 1000 to about 1500 repeat units. The weight average molecular weight is about 90,000 to about 140,000, with the polydispersity index between about 1.5 to about 3.0.

In addition to acrylic polymers, fibers and films, the antimicrobial polymers having the functional monomer of Formula I can be textiles. Textiles also include manufactured fibers based upon natural organic polymers such as, rayon, lyocell, acetate, triacetate and azlon. Textiles suitable for use in the present invention include synthetic organic polymers which include, but are not limited to, acrylic, aramid, nylon, olefin, polyester, spandex, vinyon, vinyl and graphite. Textiles also include inorganic substances such as glass, metallic and ceramic.

## III. Methods of Making Antimicrobial Polymers and Uses

In certain embodiments, the present invention provides a method for making an antimicrobial polymer composition as claimed in any preceding claim, said method comprising:

finishing or dyeing a polymer in an aqueous solution comprising a quaternary ammonium salt, wherein said polymer has a functional monomeric unit with an antimicrobial agent to form a polymer having said antimicrobial agent attached thereto;

wherein said functional monomeric unit comprises a member selected from the group consisting of a carboxylate group, a sulfonate group, an alkoxide group, a phosphate group and a phosphonate group; and
wherein said antimicrobial agent is a quaternary ammonium salt. As used herein, the terms "finishing" and "dyeing" are used interchangeably. However, "finishing" preferably refers to quaternary ammonium salts and "dyeing" preferably refers to basic dyes.

1. Effect of finishing temperature

preferably, the polymer or fiber used in the composition and methods of the present invention are synthetic organic polymers such as acrylic fibers. Acrylic fibers containing mostly amorphous structures can be finished or dyed with antimicrobial agents such as cationic dyes. In certain aspects, the dyeing temperature should be considered as dye molecules can enter into the polymers more readily at close to, or above the polymer's glass transition temperature (Tg). This is the temperature at which polymer molecules change from a glassy state to a rubbery state thus opening up more voids for penetration of chemicals.

Fig. 2 illustrates a representation of an effect of temperature on the uptake or exhaustion of an antimicrobial agent (*e.g.* cetylpyridinium chloride (CPC)). The diagram is merely an illustration and should not limit the scope of the claims herein. One of ordinary skill in the art will recognize other variations, modifications, and alternatives to the reaction conditions.

As shown in this embodiment, uptake of this particular antimicrobial agent occurred more rapidly when the temperature reached about 75 °C and gradually improved to about 20% at about 75 °C to about 85 °C, but increased to 80% at about 85 °C to about 100 °C, which is above the reported Tg of acrylics (*see,* Vigo, T.L., *Textile processing and properties, Elsevier,* p124 (1997)). In this particular instance, it appears as if the opening-up of the polymer structures began at about 75 °C and increased as the temperature increased, consequently, the uptake of the agent started to pick up and then increased at a temperature above about 85 °C. In certain aspects, the processes of the present invention are preferably carried-out at temperatures at, or near the Tg for the particular polymer under consideration.

2. Effect of antimicrobial concentration

In certain aspects, adsorption of the antimicrobial agents by the fibers depends in part on the antimicrobial agent's concentration in solution. In certain instances, increased rates of adsorptions occur at higher concentrations. Figs. 3A-B illustrate an effect of the concentration of two antimicrobial agents on their uptake. The diagrams are merely illustrations and should not limit the scope of the claims herein. One of ordinary skill in the art will recognize other variations, modifications, and alternatives to reaction conditions.

In certain instances, increasing concentrations of the finishing agents will raise the uptake rates on fibers such as acrylic fibers. Figs. 3A-B show the exhaustion patterns of (A) cetylpyridinium chloride and (B) benzyldimethylhexadecyl ammonium chloride at different concentrations. The maximum exhaustion of CDC was much higher than that of BDHAC at the same concentration of the finishing bath, and the uptake rates of the salts at higher concentrations decreased, particularly at about 4%. Exhaustion of the CPC by the fibers at different concentrations reached saturation after about 60 minutes of reaction time, but that of BDHAC could not become saturated at higher finishing concentrations. In certain instances, increasing the concentration from 1% to 2% does not vary the final exhaustion of the salts significantly, indicating increased uptake on the fibers. In certain aspects, at 4% concentration, the uptake of the salts reduced to about a half of the uptake at 2% concentration.

Without being bound by any particular theory, it is believed that cetylpyridinium chloride (CPC) is relatively smaller in size than benzyldimethylhexadedyl ammonium chloride (BDHAC), and is thus relatively easier to enter polymers (*e.g.* acrylic polymers) and interact with internal sulfonate groups. As a result of the easy adsorption, cetylpyridinium chloride can reach maximum adsorption quickly, which can be observed as saturation of the uptake after 60 minutes finishing time. High finishing temperature improves the adsorption of BDHAC on polymers (*e.g.* acrylics) due to its increased size relative to CPC. Concentration increase from 1% to 2% in the finishing bath improves adsorption.

Without being bound by any particular theory, it is believed that when the concentration reached 4%, increased quaternary ammonium salts might exceed available reactive groups on the fibers, resulting in a reduction of uptake rates for both salts. Since at 4% of concentration the fibers could uptake about half of what were exhausted at 2% of concentration, about 2% concentration is preferable.

3. Effect of pH condition

Figs. 4A-B illustrate pH effects on the uptake of two antimicrobial agents. The diagrams are merely illustrations and should not limit the scope of the claims herein. One of ordinary skill in the art will recognize other variations, modifications, and alternatives to the reaction conditions.

It can be observed that in the range of varied pH values of about 3.5 to about 7.0 the uptake of the two quaternary ammonium salts were almost unchanged, indicating in certain instances that the finishing process is less sensitive to pH conditions within the range of about 3.5 about 7.0. In certain preferred instances, the cationic dyeing of polymers (*e.g.* acrylics) is carried out in neutral or acidic conditions.

4. Durability of the finished acrylic fabrics

Fig. 5 illustrates a representation of durability of an antimicrobial agent on a fabric. The diagram is merely an illustration and should not limit the scope of the claims herein. One of ordinary skill in the art will recognize other variations, modifications, and alternatives.

In certain aspects, processes of the present invention provide for control of the exhaustion of the antimicrobial agent on fibers (*e.g.* quaternary salts on acrylics). With the quaternary ammonium salts bound to polymers, the finished acrylics exhibit durable antimicrobial properties. In Figure 5, the antimicrobial testing results from three quaternary ammonium salts, including cetylpyridinium chloride, benzyldimethylhexadedyl ammonium chloride, and dodecyltrimethyl ammonium bromide are shown. The inhibition of microorganisms by the quaternary cationic species displays about 70-80% of reduction to *Escherichia coli* upon a contact time of 18 hours. The durability of the functions was excellent with almost no reduction after 10 times of repeated Launder-Ometer washes. In certain instances, strong interactions (*e.g.* ionic) between the antimicrobial agent and the functional monomer (*e.g.* cationic salt and anionic sulfonate group) attribute to such a durable antimicrobial function. In is thus apparent that the interactions between the antimicrobial agent and the polymers (*e.g.* ionic) can be employed in antimicrobial finishing of fabrics with quaternary ammonium salts.

There are various applications for the antimicrobial polymers, of the present invention. For instance, the microbicidal textiles can provide biocidal protective clothing to personnel in the medical area as well as in the related healthcare and hygiene area. The regenerable and reusable biocidal materials can replace currently used disposable, nonwoven fabrics as medical textiles, thereby significantly reducing hospital maintenance costs and disposal fees. The microbicidal properties of the textiles of the present invention can be advantageously used for women's wear, underwear, socks, and other hygienic purposes, In addition, the microbicidal properties can be imparted to carpeting materials to create odor-free and germ-free carpets. Moreover, all germ-free environments, such as required in biotechnology and pharmaceutical industry, would benefit from the use of the microbicidal textiles of the present invention to prevent any contamination from air, liquid, and solid media.

IV. Antimicrobial Polymer Combinations

In certain aspects, the polymers and compositions of the present invention can be used in combination with other antimicrobial polymers. For example, WO 00/15897, published March 23, 2000 discloses antimicrobial fabrics that are suitable for sportswear, antiodor carpets, films, toys, *etc.* In that invention, dye molecules act as bridges to bring functional groups onto polymers and thereby serve as a point of attachment for the biocidal agents.

In another embodiment, the polymers of the present invention are used in combination with heterocyclic vinylic compounds that can be used to form biocidal polymers as disclosed in U.S. Patent Application 09/535,348 filed March 24, 2000, and entitled N-Halamine Vinyl Compounds and their Polymer Biocides, by Sun, *et al.*. As disclosed therein, the polymers generated can be used alone or can be grafted onto textiles, fabrics and polymers. The polymers are readily converted to N-halamine structures on exposure to a halogen source such as commercially available chlorine bleach. The N-halamine derivatives exhibit potent antibacterial properties against microorganisms.

In certain embodiments, the polymers of the present invention are reacted with a compound as disclosed in U.S. Patent Application 09/535,348 having the formula

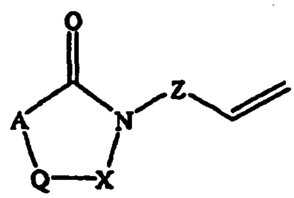

**II**

wherein A is a member selected from the group consisting of NH, N-$R^{12}$ and $CR^5R^6$, wherein $R^{12}$ is a halogen;
$R^5$ and $R^6$, are each independently selected from the group consisting of optionally substituted ($C_1$-$C_6$)alkyl, optionally substituted ($C_2$-$C_6$)alkenyl, optionally substituted ($C_2$-$C_6$)alkynyl, optionally substituted cycloalkyl, optionally substituted ($C_1$-$C_6$)alkoxy, optionally substituted aryl and optionally substituted heteroaryl;
or, $R^5$ and $R^6$ and the carbon to which they are bound join to form an optionally substituted carbocyclic or optionally substituted heterocyclic ring;
Q is a member selected from the group consisting of C(O), NH, N-$R^{13}$ and $CR^7R^8$, wherein $R^{13}$ is a halogen;
$R^7$ and $R^8$, are each independently selected from the group consisting of optionally substituted ($C_1$-$C_6$)alkyl, optionally

substituted ($C_2$-$C_6$)alkenyl, optionally substituted ($C_2$-$C_6$)alkynyl, optionally substituted cycloalkyl, optionally substituted ($C_1$-$C_6$)alkoxy, optionally substituted aryl and optionally substituted heteroaryl;
or, $R^7$ and $R^8$ and the carbon to which they are bound join to form an optionally substituted carbocyclic or optionally substituted heterocyclic ring;
X is a member selected from the group consisting of C(O), C(O)-$NR^9$ and $CR^{10}R^{11}$, wherein $R^9$ is a member selected from the group consisting of hydrogen, halogen, optionally substituted ($C_2$-$C_6$)alkenyl and optionally substituted ($C_1$-$C_6$) alkyl;
$R^{10}$ and $R^{11}$, are each independently selected from the group consisting of optionally substituted ($C_1$-$C_6$)alkyl, optionally substituted ($C_2$-$C_6$)alkenyl, optionally substituted ($C_2$-$C_6$)alkynyl, optionally substituted cycloalkyl, optionally substituted ($C_1$-$C_6$)alkoxy, optionally substituted aryl and optionally substituted heteroaryl;
or, $R^{10}$ and $R^{11}$ and the carbon to which they are bound join to form an optionally substituted carbocyclic or optionally substituted heterocyclic ring; and
Z is a member selected from the group consisting of optionally substituted ($C_1$-$C_3$)alkylene, C(O), or a single bond, with a vinyl monomer in a reaction mixture thereby chemically modifying the polymer. As used herein "chemical modification" includes grafting the monomers alone or as a copolymer onto existing natural or synthetic polymers in the presence of at least one other existing vinyl monomer. The polymerization and chemical modification reactions can be initiated by thermal or radiation method, or the combinations thereof, optionally in the presence of initiators. The resultant grafted polymers can be used as plastics, rubbers, polymeric materials, paints, surface coatings, adhesives, etc., in the form of bulk, films/membranes, powder, solutions, gels, *etc.*

The biocidal polymer combinations of the present invention are effective against all microorganisms. Such microorganisms include, for example, bacteria, protozoa, fungi, viruses and algae. Moreover, the biocidal polymers described herein can be employed in a variety of disinfecting applications, such as water purification. They will be of importance in controlling microbiological contamination or growth of undesirable organisms in the medical and food industries. In addition, they can be used as preservatives and preventatives against microbiological contamination in paints, coatings, and on surfaces.

The invention will be described in greater detail by way of specific examples. The following examples are offered for illustrative purposes, and are not intended to limit the invention in any manner.

## V. Examples

### 1. Materials

Orlon (#864) (commercially available from Testfabrics Inc.) was thoroughly scoured with AATCC standard detergent 124 before use. Basic dyes were supplied by Aldrich. The quaternary ammonium salts employed in this study were cetylpyridinium chloride (commercially available from Aldrich), benzyldimethylhexadecylammonium chloride (commercially available from Acros) and dodecyltrimethyl ammonium bromide (commercially available from Aldrich), with structures shown in Figure 1A.

### 2. Finishing

Antibacterial finishing baths were prepared by dissolving quaternary ammonium salts in distilled water. The pH values of the solutions were adjusted with acetic acid, and sodium sulfate (0 -5 % owf) was added as a leveling agent. The liquor ratio was 100:1. The fabrics were immersed in the finishing bath, and the temperature of the finishing bath was raised at a rate of 1 °C/min. to boiling. Then the finishing continued at boiling for a duration of 90 minutes, during which aliquots of finishing solution were taken from the finishing bath at finishing times of 0, 15,30,45, 60,75 and 90 min. respectively, for monitoring adsorption processes of the salts. Absorbance of these solutions was then measured at the wavelength of maximum absorption ($\lambda_{max}$) using a UV-VIS spectrophotometer (Hitachi U-2000), with the $\lambda_{max}$ values of cetylpyridinium chloride and benzyldimethylhexadedylammonium chloride at 265nm and 262nm, respectively. The percentage uptake of finishing agent was calculated according to Equation 1:

$$\text{Uptake (\%)} = (Ao - A\,t)/\,Ao \times 100 \quad (1)$$

where Ao and At represent the absorbance of the finishing solution before the finishing started and after a finishing time of t, respectively. To examine the effect of temperature in the antimicrobial finishing, when the desired finishing temperature was reached, the finishing bath was kept at this temperature for 20 min., and an aliquot of the finishing bath was taken, and then the temperature was then raised to the next higher one.

3. Basic dyeing and finishing

The Orlon fabrics were treated in a bath containing both 1-4% owf of a basic dye and 2-4% owf of a quaternary ammonium salt. The pH values of the finishing baths were adjusted to 4.5 with acetic acid. The liquor ratio used was 100:1. The fabrics were immersed in the dyeing and finishing bath, and the temperature of the finishing bath was raised at a rate of 1°C/min. to boiling. Then the process continued at boiling for 90 minutes. Aliquots of finishing solution were taken from the finishing bath before and after treated fabric. The absorbance of these solutions was then measured at the wavelength of maximum absorption ($\lambda_{max}$) using a UV-VIS spectrophotometer(Hitachi U-2000), with the $\lambda_{max}$ values of cetylpyridinium chloride and Basic Blue 69 at 265nm and 582nm, respectively. The percentage uptake of dye and quaternary ammonium salt were calculated according to the Equation 1.

4. Deterpency test

The finished fabric was washed in a Launder-Ometer, according to AATCC Test Method 61-1994 to evaluate the washing durability. One cycle of Launder-Ometer washing test by this AATCC Test Method is considered equivalent to five machine-washes in a home laundry.

5. Antibacterial test

The antimicrobial properties were quantitatively evaluated against *Escherichia coli,* ATCC 2666, a gram-negative bacterium, according to AATCC Test Method 100-1993. Circular fabric swatches (about one gram) were challenged with 1.0 (0.1mL of bacterial inoculum) in a 250 mL container. The inoculum was a nutrient broth culture containing 1.0 X $10^4$-$10^6$ /mL colony forming units (CFU) of bacteria. After test and control swatches had been contacted in with bacteria for 18 hours, 100 mL of sterilized distilled water was poured into the container, which was then vigorously shaken, and the supernatant was diluted to $10^1$, $10^2$, $10^3$ and $10^4$. The diluted solution aliquots were plated on a nutrient agar and incubated for 18 hours at 37 °C. Viable colonies of bacteria on the agar plate were counted. The reduction in numbers of bacteria was calculated using the following equation.

$$\text{Reduction rate (\%)} = (A-B)/A \times 100$$

Where A is the numbers of bacterial colonies from untreated fabrics and B is the numbers of bacterial colonies from treated fabrics.

**Example 1**

This Example illustrates the effect of temperature on exhaustion of antimicrobial agent.

A piece of acrylic (Orlon) fabric was treated in a bath containing 1% cetylpyridinium chloride (CPC) (Aldrich) on weight of fabric (owf), and 5% of sodium sulfate (SS). The pH value of the finishing bath was adjusted to 5 with acetic acid. The liquor ratio was 100:1. The fabric was immersed in the finishing bath, and the temperature of the finishing bath was raised at a rate of 1°C/min. to boiling. Then the finishing continued at boiling for 90 minutes. Aliquots of finishing solution were taken from the finishing bath before and after the treatment. The absorbance of these solutions was then measured at the wavelength of maximum absorption ($\lambda_{max}$) using a UV-VIS spectrophotometer (Hitachi U-2000). The percentage uptake of the quaternary ammonium salt was calculated according to Equation 1. The results are shown in Figure 2.

**Example 2**

This Example illustrates the effect of uptake of CPC and BDHAC on Orlon.

Orlon (#864) fabrics were treated in a bath containing 1, 2, 4% cetylpyridinium chloride (CPC) or benzyldimethylhexyldecyl ammonium chloride (BDHDA) (Aldrich) on weight of fabric (owf), and 5% of sodium sulfate (SS). The pH values of the finishing baths were adjusted to 4.5 with acetic acid. The liquor ratio was 100:1. The fabrics were immersed in the finishing bath, and the temperature of the finishing bath was raised at a rate of 1°C/min. to boiling. Then the finishing continued at boiling for 90 minutes. Aliquots of finishing solution were taken from the finishing bath before and after treatment. The absorbance of these solutions was then measured at the wavelength of maximum absorption ($\lambda_{max}$) using a UV-VIS spectrophotometer (Hitachi U-2000). The percentage uptake of the quaternary ammonium salts was calculated according to Equation 1. The results are shown in Figures 3A-B.

**Example 3**

This Example illustrates add-on of CPC on different cationic dyeable fabrics.

Orlon(#864), Acrylan (#981), and cationic dyeable polyester(#763H), purchased from Testfabrics Inc., were thoroughly scoured with AATCC standard detergent 124 before use. The fabrics were treated in a bath containing 4% owf of Cetylpyridinium chloride (CPC) (Aldrich) and 5% owf of sodium sulfate. The pH values of the finishing baths were adjusted to 4.5 with acetic acid. The liquor ratio was 100:1. The add-ons of CPC on the fabrics after rinsing are shown in Figure 6.

**Example 4**

This Example illustrates add-on of the salts on acrylic fabrics after rinsing and washing.

Orlon fabrics were treated in a bath containing 4% Cetylpyridinium chloride. Benzyldimethylhexyldecyl ammonium chloride (BDHDA) (Aldrich), and trimethyldodecyl ammonium bromide (DTAB), (Aldrich) on weight of fabric (owf). The pH values of the finishing baths were adjusted to 4.5 with acetic acid. The liquor ratio was 100:1. The fabrics were immersed in the finishing bath, and the temperature of the finishing bath was raised at a rate of 1°C/min. to boiling. Then the finishing continued at boiling for 90 minutes. The add-ons of the salts on the fabrics after rinsing were listed in Figure 7.

**Example 5**

This Example illustrates durability of antimicrobial functions of finishing acrylic fabrics.

The fabrics listed in Figure 6 were then carried out for antimicrobial tests after repeated washing. The finished fabric was washed in a Launder-Ometer, according to AATCC Test Method 61-1994 to evaluate the washing durability. One gram of the fabric was put in a solution of 0.225 gram of AATCC detergent 124 in 150 mL of $H_2O$ in a container having 50 stainless steel balls inside, and the container was sealed and put into the launder-Ometer at room temperature. The washing time was 45 minutes, and then the fabric was rinsed in distilled water, and air-dried. One cycle of Launder-Ometer washing test by this AATCC Test Method is considered equivalent to five machine-washes in a home laundry.

The antimicrobial properties of the fabrics were quantitatively evaluated against *Escherichia coli,* ATCC 2666, a gram-negative bacterium, according to AATCC Test Method 100-1993. Circular fabric swatches (about one gram) were challenged with 1.0(0.1mL of bacterial inoculum in a 250 mL container. The inoculum was a nutrient broth culture containing 1.0(104-106/mL colony forming units (CFU) of bacteria. After test and control swatches had been contacted in with bacteria for 18 hours, 100 mL of sterilized distilled water was poured into the container, which was then vigorously shaken, and the supernatant was diluted to $10^1$, $10^2$, $10^3$ and $10^4$. The diluted solution aliquots were plated on a nutrient agar and incubated for 18 hours at 37°C. Viable colonies of bacteria on the agar plate were counted. The reduction in numbers of bacteria was calculated using the reduction rate equation set forth above. The results are shown in Figure 5.

**Example 6**

This Example illustrates antimicrobial functions of acrylics finished by basic dyes.

The Orlon fabrics were treated in a bath containing a basic dye at 1-% owf or 1-% owf of CPC. The basic dyes were Basic red 9 (R9), basic orange 14 (O14), basic blue 9 (B9), basic blue 69 (B69), and basic blue 22 (B22). The pH values of the finishing baths were adjusted to 4.5 with acetic acid. The liquor ratio used was 100:1. The fabrics were immersed in the dyeing and finishing bath, and the temperature of the finishing bath was raised at a rate of 1°C/min. to boiling. Then the process continued at boiling for 90 minutes.

The antimicrobial properties of the fabrics were quantitatively evaluated against *Escherichia coli,* ATCC 2666, a gram-negative bacterium, according to AATCC Test Method 100-1993. The results are shown in Figure 8.

**Example 7**

This Example illustrates antimicrobial functions of acrylics treated by a mixture of a basic dye and CPC.

The Orlon fabrics were treated in a bath containing a mixture of a basic dye (2% owf) and 2% owf of CPC. The basic dyes were basic red 9 (R9), basic orange 14 (O14 ), basic blue 9 (B9), basic blue 69 (B69), and basic blue 22 (B22). The pH values of the finishing baths were adjusted to 4.5 with acetic acid. The liquor ratio used was 100:1. The fabrics were immersed in the dyeing and finishing bath, and the temperature of the finishing bath was raised at a rate of 1°C/min. to boiling. Then the process continued at boiling for 90 minutes. The antimicrobial properties of the fabrics were quantitatively evaluated against *Escherichia coli,* ATCC 2666, a gram-negative bacterium, according to AATCC Test Method 100-1993. The results are shown in Figure 9.

**Example 8**

This Example illustrates the durability of antimicrobial functions of dyed and finished acrylics.

The Orlon fabrics were treated in a bath containing a mixture of a basic dye and CPC in varied ratios listed in Table A. The basic dye used was basic blue 69. The pH values of the finishing baths were adjusted to 4.5 with acetic acid. The liquor ratio used was 100:1. The fabrics were immersed in the dyeing and finishing bath, and the temperature of the finishing bath was raised at a rate of 1 °C/min. to boiling. Then the process continued at boiling for 90 minutes. The finished fabric was washed in a Launder-Ometer, according to AATCC Test Method 61-1994 to evaluate the washing durability.

The antimicrobial properties of the fabrics were quantitatively evaluated after each washing against *Escherichia coli,* ATCC 2666, a gram-negative bacterium, according to AATCC Test Method 100-1993. The results are shown in Table 1.

Table 1 Durability of antimicrobial functions of dyed and finished acrylics

| Washing time | Dye to Quaternary ammonium salt ratio | | | | | |
|---|---|---|---|---|---|---|
| | 1:4 | 2:4 | 4:4 | 2:2 | 0:2 | 0:4 |
| 2 | 75.4 | 77.9 | 80.4 | 82.8 | 85.3 | 75.4 |
| 3 | 86.1 | 78.2 | 72.5 | 87.9 | 87.1 | 88.6 |
| 4 | 93.1 | 85.5 | 88.6 | 89.5 | 83.7 | 87.3 |
| 5 | 80.8 | 81.2 | 81.7 | 79.3 | 71.8 | 77 |
| 6 | 90 | 85.6 | 84.3 | 82.3 | 79.4 | 90 |
| 7 | 86.8 | 86.8 | 87.3 | 82.9 | 85.4 | 86 |
| 8 | 75.4 | 66.5 | 72.1 | 68.2 | 65.1 | 71.5 |
| 9 | 87.4 | 88.1 | 87.8 | 84.8 | 81 | 88.1 |
| 10 | 81.6 | 79.9 | 81.6 | 74.2 | 82.9 | 83.9 |

**Example 9**

This Example illustrates the color fastness grades of the dyed and finished acrylics.

The Orlon fabrics were treated in a bath containing a mixture of a basic dye and CPC in varied ratios listed in Table 2. The basic dye used was basic blue 69. The pH values of the finishing baths were adjusted to 4.5 with acetic acid. The liquor ratio used was 100:1. The fabrics were immersed in the dyeing and finishing bath, and the temperature of the finishing bath was raised at a rate of 1°C/min. to boiling. Then the process continued at boiling for 90 minutes. The finished fabric was washed in a Launder-Ometer, according to AATCC Test Method 61-1994 and then the color of the fabric sample was evaluated according to an AATCC Evaluation Procedure One to evaluate color fastness of the dyed and finished fabrics.

Table 2 Color Fastness grades of the dyed and finished acrylics

| Basic blue 69:CPC | Color difference after 10 LO washing | Color Fastness Grade |
|---|---|---|
| 1:4 | 0.66 | 4-5 |
| 2:4 | 1.05 | 4 |
| 4:4 | 1.29 | 4 |
| 2:2 | 0.69 | 4-5 |

**Claims**

1. An antimicrobial polymer composition, said composition comprising a polymer having at least one functional monomeric unit of the formula:

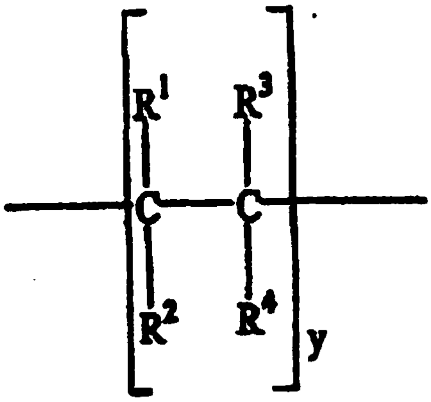

I

wherein:

$R^1$, $R^2$, and $R^3$ are each independently, a member selected from the group consisting of hydrogen, halogen, hydroxyl, cyano, $(C_{1-}C_6)$ alkyl, $(C_2-C_6)$ alkenyl, $(C_1-C_6)$ alkoxy, $(C_{1-}C_6)$alkylcarbonyl, $(C_1-C_6)$alkylcarboxyl, aldehydo, amido, aryl and heterocyclyl;
$R^4$ is a functional group moiety selected from the group consisting of $-CO_2^-X^+$, $-SO_3-X^+$, $-O^-X^+$, $-PO_4^{-2}ZX^+$ and $-PO_3^{-2}ZX^+$;
X is a quaternary ammonium ion selected from the group consisting of a cetylpyridinium ion, a benzyldimethylhexadecylammonium ion, a N-(3-chloro-2-hydroxypropyl)-N,N-dimethyldodecylammonium ion, a 1,3-Bis-(N, N-dimethyldodecylammonium ion)-2-propanol, a N-(1-(2,3-dioleoyloxy)propyl)-N,N,N-trimethylammonium ion, a N-(1-(2,3-dioleyloxy)propyl)-N,N,N-trimethylammonium ion, a N,N-dioleyl-N,N-dimethylammonium ion, a 1,2-dioleoyloxy-3-(N,N,N-trimethylamino)propane ion and mixtures thereof; and
Z is a member selected from the group consisting of hydrogen and an alkaline earth metal; and
y is an integer from 1 to 250 inclusive.

**2.** The composition according to Claim 1, wherein said polymer is a member selected from the group consisting of a fiber, a film and a textile.

**3.** The composition according to Claim 2, wherein said fiber is a member selected from the group consisting of a fiber from plants, a polymer from animals, a natural organic polymer, a synthetic organic polymer and an inorganic substance.

**4.** The composition according to Claim 3, wherein said synthetic organic polymer is selected from the group consisting of an acrylic polymer, an aramid polymer, a nylon polymer, an olefin polymer, a spandex polymer, a vinyon polymer, a vinyl polymer, and cationic dyeable polyester.

**5.** The composition according to any preceding claim, wherein said functional monomeric unit is a negatively charged monomeric unit comprising a sulfonated monomer.

**6.** The composition according to Claim 5, wherein said sulfonated monomer is selected from the group consisting of sodium styrenesulfonate, sodium methyallyl sulfonate and sodium sulfophenyl methallyl ether.

**7.** The composition according to any preceding claim wherein said quaternary ammonium salt is a member selected from the group consisting of a cetylpyridinium ion and a benzyldimethylhexadecylammonium ion, and mixtures thereof.

**8.** The composition according to any preceding claim wherein said polymer is a textile.

**9.** The composition according to any one of Claims 1 to 7, wherein said polymer is a long-chain synthetic acrylic polymer or fiber comprising at least 35% by weight of acrylonitrile units.

**10.** The composition according to Claim 3, wherein said fiber from plants is selected from the group consisting of

cellulose, cotton, linen, hemp, jute and ramie.

**11.** The composition according to Claim 3, wherein said polymer from animals is selected from the group consisting of wool, mohair, vicuna and silk.

**12.** The composition according to Claim 3, wherein said organic polymer is selected from the group consisting of rayon, lyocell, acetate, triacetate, and azlon.

**13.** A method for making an antimicrobial polymer composition as claimed in any preceding claim, said method comprising:

finishing or dyeing a polymer in an aqueous solution comprising a quaternary ammonium salt, wherein said polymer has a functional monomeric unit with an antimicrobial agent to form a polymer having said antimicrobial agent attached thereto;

wherein said functional monomeric unit comprises a member selected from the group consisting of a carboxylate group, a sulfonate group, an alkoxide group, a phosphate group and a phosphonate group; and
wherein said antimicrobial agent is a quaternary ammonium salt.

**14.** The method according to Claim 13, wherein said polymer is a member selected from the group consisting of a fiber, a film and a textile.

**15.** The method according to Claim 14, wherein said fiber is a member selected from the group consisting of a fiber from plants, a polymer from animals, a natural organic polymer, a synthetic organic polymer and an inorganic substance.

**16.** The method according to Claim 15, wherein said synthetic organic polymer is selected from the group consisting of an acrylic polymer and a cationic dyeable polyester.

**17.** The method according to any one of Claims 13 to 16, wherein said functional monomeric unit is a negatively charged monomeric unit comprising a sulfonated monomer.

**18.** The method according to Claim 17, wherein said sulfonated monomer is selected from the group consisting of sodium styrenesulfonate, sodium methyallyl sulfonate and sodium sulfophenyl methallyl ether.

**19.** The method according to any one of Claims 13 to 18, wherein said quaternary ammonium salt is a member selected from the group consisting of a cetylpyridinium ion and a benzyldimethylhexadecylammonium ion.

**20.** The method according to any of Claims 13 to 19, wherein said polymer is a textile.

**21.** The method according to any of Claims 13 to 20, wherein finishing or dyeing said polymer occurs at close to or above the glass transition temperature ($T_g$) of said polymer.

**22.** The method according to any of Claims 13 to 21, wherein said antimicrobial agent is in a solution having a pH within the range of 3.5 to 7.0.

## Revendications

**1.** Une composition polymère antimicrobienne, ladite composition comportant un polymère ayant au moins un motif monomère fonctionnel de formule :

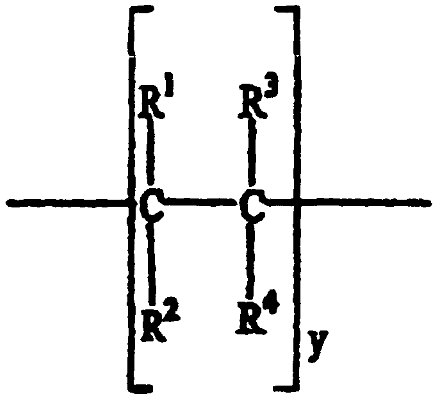

dans laquelle :

$R^1$, $R^2$, et $R^3$ sont chacun indépendamment, un élément sélectionné dans le groupe consistant en hydrogène, halogène, hydroxyle, cyano, alkyle ($C_1$-$C_6$), alkényle ($C_2$-$C_6$), alkoxy ($C_1$-$C_6$), alkylcarbonyle ($C_1$-$C_6$), alkylcarboxyle ($C_1$-$C_6$), aldéhyde, amide, aryle et hétérocyclyl ;
$R^4$ est un fragment de groupe fonctionnel sélectionné dans le groupe consistant en $-CO_2^-X^+$, $-SO_3^-X^+$, $-O^-X^4$, $-PO_4^{-2}ZX^+$ et $-PO_3^{-2}Z^+$ ;
X est un ion ammonium quaternaire sélectionné dans le groupe consistant en ion cétylpyridinium, ion benzyldiméthylhexadécylammonium, ion N-(3-chloro-2-hydroxypropyl)-N,N-diméthyldodécylammonium, 1,3-Bis-(ion N,N-diméthyldodécylammonium)-2-propanol, ion N-(1-(2,3-dioléoyloxy)propyl)-N,N,N-triméthylammoniumn, ion N-(1-(2,3-dioléyloxy)propyl)-N,N,N-triméthylammonium, ion N,N-dioléyl-N,N-diméthylammonium, ion 1,2-dioléoyloxy-3-(N,N,N-triméthylamino)propane et mélanges de ceux-ci ; et
Z est un élément sélectionné dans le groupe consistant en hydrogène et un métal alcalinoterreux ; et
y est un nombre entier allant de 1 à 250 inclus.

**2.** La composition selon la revendication 1, dans laquelle ledit polymère est un élément sélectionné dans le groupe consistant en fibre, film et textile.

**3.** La composition selon la revendication 2, dans laquelle ladite fibre est un élément sélectionné dans le groupe consistant en fibre provenant de plantes, polymère provenant d'animaux, polymère organique naturel, polymère organique synthétique et substance inorganique.

**4.** La composition selon la revendication 3, dans laquelle ledit polymère organique synthétique est sélectionné dans le groupe consistant en polymère acrylique, polymère aramide, polymère nylon, polymère oléfine, polymère spandex, polymère vinyon, polymère vinyl et polyester cationique pouvant être teint.

**5.** La composition selon n'importe quelle revendication précédente, dans laquelle ledit motif monomère fonctionnel est un motif monomère à charge négative comportant un monomère sulfoné.

**6.** La composition selon la revendication 5, dans laquelle ledit monomère sulfoné est sélectionné dans le groupe consistant en styrènesulfonate de sodium, méthylallyl sulfonate de sodium et éther méthylallyl sulfophényl de sodium.

**7.** La composition selon n'importe quelle revendication précédente dans laquelle ledit sel d'ammonium quaternaire est un élément sélectionné dans le groupe consistant en ion cétylpyridinium et ion benzyldiméthylhexadécylammonium, et des mélanges de ceux-ci.

**8.** La composition selon n'importe quelle revendication précédente dans laquelle ledit polymère est un textile.

**9.** La composition selon n'importe laquelle des revendications 1 à 7, dans laquelle ledit polymère est un polymère ou fibre acrylique synthétique à longue chaîne comportant au moins 35 % en poids de motifs acrylonitrile.

**10.** La composition selon la revendication 3, dans laquelle ladite fibre provenant de plantes est sélectionnée dans le groupe consistant en cellulose, coton, lin, chanvre, jute et ramie.

**11.** La composition selon la revendication 3, dans laquelle ledit polymère provenant d'animaux est sélectionné dans le groupe consistant en laine, mohair, vigogne et soie.

**12.** La composition selon la revendication 3, dans laquelle ledit polymère organique est sélectionné dans le groupe consistant en rayonne, lyocell, acétate, triacétate, et azlon.

**13.** Une méthode de fabrication d'une composition polymère antimicrobienne tel que revendiqué dans n'importe quelle revendication précédente, ladite méthode comportant :

le finissage ou la teinture d'un polymère dans une solution aqueuse comportant un sel d'ammonium quaternaire, dans laquelle ledit polymère a un motif monomère fonctionnel avec un agent antimicrobien pour former un polymère ayant ledit agent antimicrobien attaché à celui-ci ;

dans laquelle ledit motif monomère fonctionnel comporte un élément sélectionné dans le groupe consistant en groupe carboxylate, groupe sulfonate, groupe alcoxyde, groupe phosphate et groupe phosphonate ; et
dans laquelle ledit agent antimicrobien est un sel d'ammonium quaternaire.

**14.** La méthode selon la revendication 13, dans laquelle ledit polymère est un élément sélectionné dans le groupe consistant en fibre, film et textile.

**15.** La méthode selon la revendication 14, dans laquelle ladite fibre est un élément sélectionné dans le groupe consistant en fibre provenant de plantes, polymère provenant d'animaux, polymère organique naturel, polymère organique synthétique et substance inorganique.

**16.** La méthode selon la revendication 15, dans laquelle ledit polymère organique synthétique est sélectionné dans le groupe consistant en polymère acrylique et polyester cationique pouvant être teint.

**17.** La méthode selon n'importe laquelle des revendications 13 à 16, dans laquelle ledit motif monomère fonctionnel est un motif monomère à charge négative comportant un monomère sulfoné.

**18.** La méthode selon la revendication 17, dans laquelle ledit monomère sulfoné est sélectionné dans le groupe consistant en styrènesulfonate de sodium, sulfonate méthylallyl de sodium et éther méthylallyl sulfophényl de sodium.

**19.** La méthode selon n'importe laquelle des revendications 13 à 18, dans laquelle ledit sel d'ammonium quaternaire est un élément sélectionné dans le groupe consistant en ion cétylpyridinium et ion benzyldiméthylhexadécylammonium.

**20.** La méthode selon n'importe lesquelles des revendications 13 à 19, dans laquelle ledit polymère est un textile.

**21.** La méthode selon n'importe lesquelles des revendications 13 à 20, dans laquelle le finissage ou la teinture dudit polymère se produit près de la température de transition vitreuse ($T_g$) dudit polymère ou au-dessus de celle-ci.

**22.** La méthode selon n'importe lesquelles des revendications 13 à 21, dans laquelle ledit agent antimicrobien est dans une solution ayant un pH dans la gamme allant de 3,5 à 7,0.

## Patentansprüche

**1.** Eine antimikrobielle Polymerzusammensetzung, wobei die **Zusammensetzung ein Polymer mit mindestens einer funktionellen** monomeren Einheit der folgenden Formel beinhaltet:

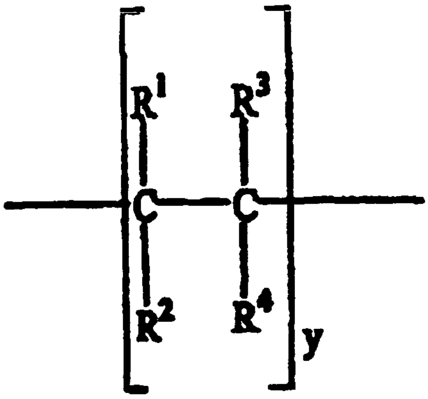

I

wobei:

$R^1$, $R^2$ und $R^3$ jeweils unabhängig ein Teil, ausgewählt aus der Gruppe, bestehend aus Wasserstoff, Halogen, Hydroxyl, Cyano, $(C_1$-$C_6)$-Alkyl, $(C_2$-$C_6)$-Alkenyl, $(C_1$-$C_6)$-Alkoxy, $(C_1$-$C_6)$-Alkylcarbonyl, $(C_1$-$C_6)$-Alkylcarboxyl, Aldehydo, Amido, Aryl und Heterocyclyl, sind;
$R^4$ ein funktioneller Gruppenanteil, ausgewählt aus der Gruppe, die aus $-CO_2^-X^+$, $-SO_3^-X^+$, $-O^-X^+$, $-PO_4^{-2}ZX^+$ und $-PO_3^{-2}ZX^+$ besteht, ist;
X ein quaternäres Ammoniumion, ausgewählt aus der Gruppe, bestehend aus einem Cetylpyridiniumion, einem Benzyldimethylhexadecylammoniumion, einem N-(3-Chlor-2-hydroxypropyl)-N,N-dimethyldodecylammoniumion, einem 1,3-Bis-(N,N-Dimethyldodecylammoniumion)-2-propanol, einem N-(1-(2,3-Dioleoyloxy)propyl)-N,N,N-trimethylammonlumion, einem N-(1-(2,3-Dioleyloxy)propyl)-N,N,N-trimethylammonlumion, einem N,N-Dioleyl-N,N-dimethylammoniumion, einem 1,2-Dioleoyloxy-3-(N,N,N-trimethylamino)propanion und Gemischen von diesen, ist; und
Z ein Teil, ausgewählt aus der Gruppe, bestehend aus Wasserstoff und einem Erdalkalimetall, ist; und
y eine ganze Zahl von 1 bis einschließlich 250 ist.

**2.** Zusammensetzung gemäß Anspruch 1, wobei das Polymer ein Teil, ausgewählt aus der Gruppe, bestehend aus einer Faser, einem Film und einem Textil, ist.

**3.** Zusammensetzung gemäß Anspruch 2, wobei die Faser ein Teil, ausgewählt aus der Gruppe, bestehend aus einer Faser von Pflanzen, einem Polymer von Tieren, einem natürlichen organischen Polymer, einem synthetischen organischen Polymer und einer anorganischen Substanz, ist.

**4.** Zusammensetzung gemäß Anspruch 3, wobei das synthetische organische Polymer aus der Gruppe, bestehend aus einem Acrylpolymer, einem Aramidpolymer, einem Nylonpolymer, einem Olefinpolymer, einem Elasthanpolymer, einem Vinyonpolymer, einem Vinylpolymer und kationisch färbbarem Polyester, ausgewählt ist.

**5.** Zusammensetzung gemäß einem der vorhergehenden Ansprüche, wobei die funktionelle monomere Einheit eine negativ geladene monomere Einheit ist, die ein sulfoniertes Monomer beinhaltet.

**6.** Zusammensetzung gemäß Anspruch 5, wobei das sulfonierte Monomer aus der Gruppe, bestehend aus Natriumstyrensulfonat, Natriummethylallylsulfonat und Natriumsulfophenylmethylallylether, ausgewählt ist.

**7.** Zusammensetzung gemäß einem der vorhergehenden Ansprüche, wobei das quaternäre Ammoniumsalz ein Teil, ausgewählt aus der Gruppe, bestehend aus einem Cetylpyridiniumion und einem Benzyldimethylhexadecylammoniumion und Gemischen von diesen, ist.

**8.** Zusammensetzung gemäß einem der vorhergehenden Ansprüche, wobei das Polymer ein Textil ist.

**9.** Zusammensetzung gemäß einem der Ansprüche 1 bis 7, wobei das Polymer ein langkettiges synthetisches Acrylpolymer oder eine langkettige synthetische Acrylfaser ist, das/die mindestens 35 Gew.-% Acrylnitrileinheiten beinhaltet.

**10.** Zusammensetzung gemäß Anspruch 3, wobei die Faser von Pflanzen aus der Gruppe, bestehend aus Cellulose, Baumwolle, Leinen, Hanf, Jute und Ramie, ausgewählt ist.

**11.** Zusammensetzung gemäß Anspruch 3, wobei das Polymer von Tieren aus der Gruppe, bestehend aus Wolle, Mohär, Vikunja und Seide, ausgewählt ist.

**12.** Zusammensetzung gemäß Anspruch 3, wobei das organische Polymer aus der Gruppe, bestehend aus Rayon, Lyocell, Acetat, Triacetat und Azlon, ausgewählt ist.

**13.** Ein Verfahren zum Herstellen einer antimikrobiellen Polymerzusammensetzung gemäß einem der vorhergehenden Ansprüche, wobei das Verfahren Folgendes beinhaltet:

Ausrüsten oder Färben eines Polymers in einer wässrigen Lösung, die ein quaternäres Ammoniumsalz beinhaltet, wobei das Polymer eine funktionelle monomere Einheit mit einem antimikrobiellen Wirkstoff aufweist, um ein Polymer zu bilden, an dem der antimikrobielle Wirkstoff hängt;

wobei die funktionelle monomere Einheit ein Teil, ausgewählt aus der Gruppe, bestehend aus einer Carboxylatgruppe, einer Sulfonatgruppe, einer Alkoxidgruppe, einer Phosphatgruppe und einer Phosphonatgruppe, beinhaltet; und
wobei der antimikrobielle Wirkstoff ein quaternäres Ammoniumsalz ist.

**14.** Verfahren gemäß Anspruch 13, wobei das Polymer ein Teil, ausgewählt aus der Gruppe, bestehend aus einer Faser, einem Film und einem Textil, ist.

**15.** Verfahren gemäß Anspruch 14, wobei die Faser ein Teil, ausgewählt aus der Gruppe, bestehend aus einer Faser von Pflanzen, einem Polymer von Tieren, einem natürlichen organischen Polymer, einem synthetischen organischen Polymer und einer anorganischen Substanz, ist.

**16.** Verfahren gemäß Anspruch 15, wobei das synthetische organische Polymer aus der Gruppe, bestehend aus einem Acrylpolymer und einem kationischen färbbaren Polyester, ausgewählt ist.

**17.** Verfahren gemäß einem der Ansprüche 13 bis 16, wobei die funktionelle monomere Einheit eine negativ geladene monomere Einheit ist, die ein sulfoniertes Monomer beinhaltet.

**18.** Verfahren gemäß Anspruch 17, wobei das sulfonierte Monomer aus der Gruppe, bestehend aus Natriumstyrensulfonat, Natriummethylallylsulfonat und Natriumsulfophenylmethylallylether, ausgewählt ist.

**19.** Verfahren gemäß einem der Ansprüche 13 bis 18, wobei das quaternäre Ammoniumsalz ein Teil, ausgewählt aus der Gruppe, bestehend aus einem Cetylpyridiniumion und einem Benzyldimethylhexadecylammoniumion, ist.

**20.** Verfahren gemäß einem der Ansprüche 13 bis 19, wobei das Polymer ein Textil ist.

**21.** Verfahren gemäß einem der Ansprüche 13 bis 20, wobei Ausrüsten oder Färben des Polymers in der Nähe von oder über der Glasübergangstemperatur ($T_g$) des Polymers stattfindet.

**22.** Verfahren gemäß einem der Ansprüche 13 bis 21, wobei sich der antimikrobielle Wirkstoff in einer Lösung mit einem pH-Wert in dem Bereich von 3,5 bis 7,0 befindet.

A

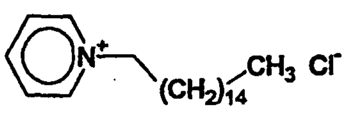

Cetylpyridinium chloride

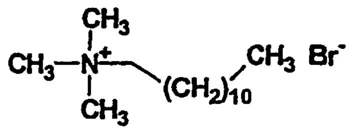

Dodecyltrimethylammonium bromide

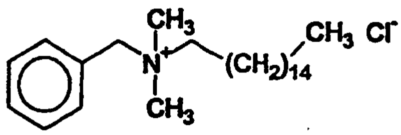

Benzyldimethylhexadecylammonium chloride

B

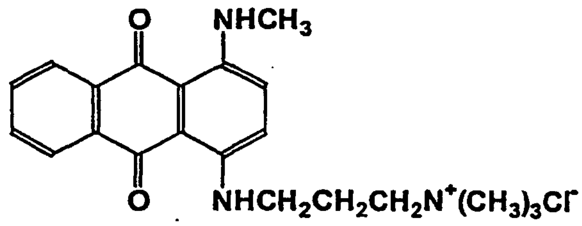

Basic Blue 22

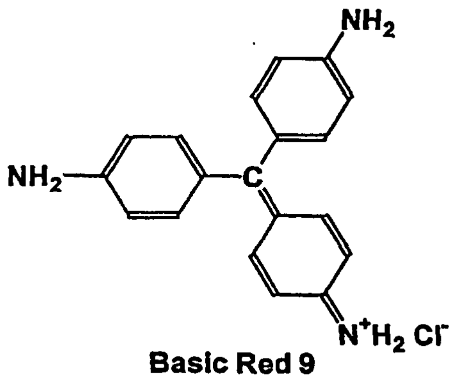

Basic Red 9

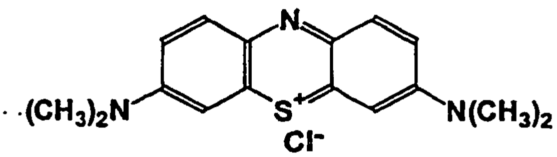

Basic Blue 9

*FIG. 1*

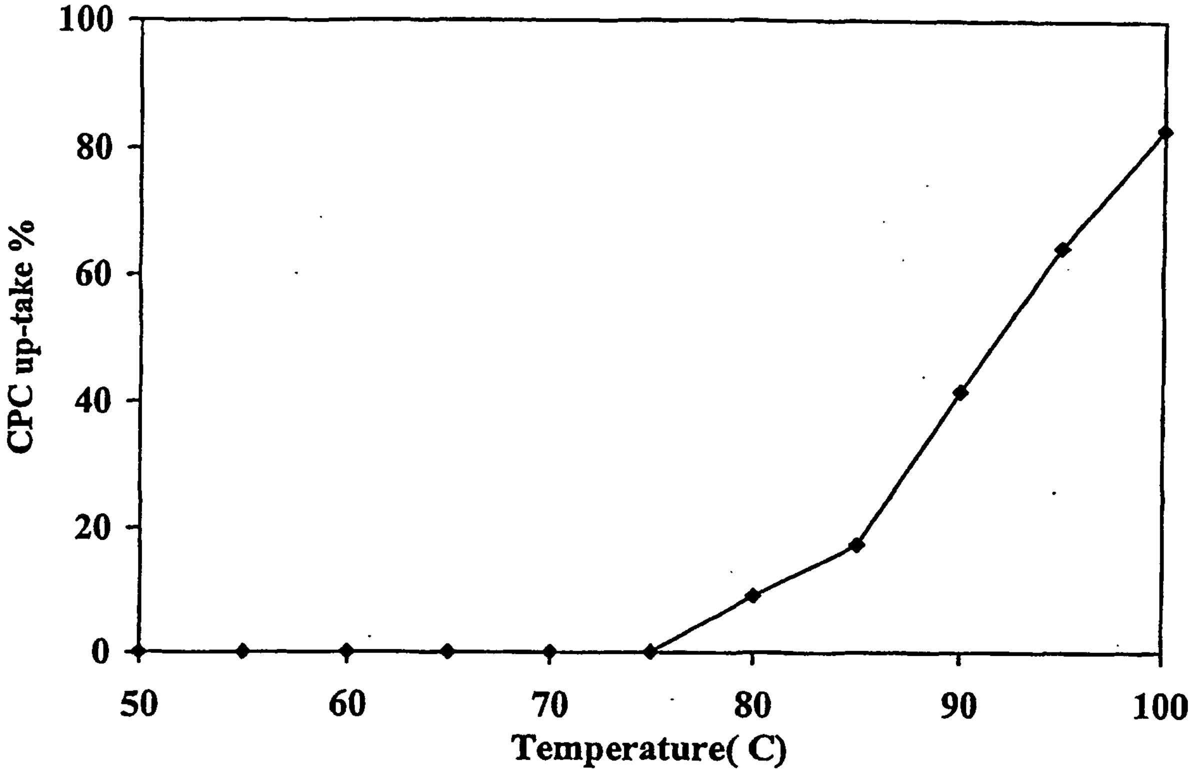


*FIG. 2*

A

B

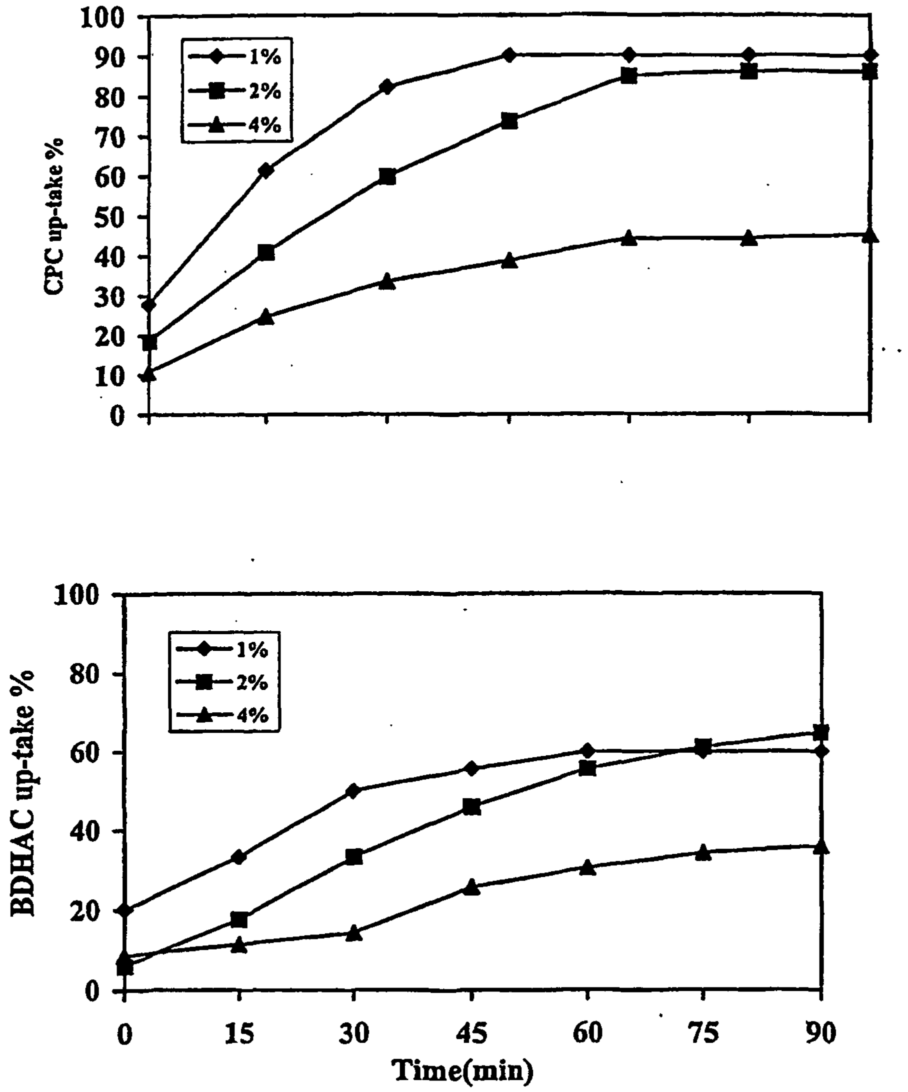
100
90
80
70
60
50
40
30
20
10
0
CPC up-take %
1%
2%
4%
100
80
60
40
20
0
BDHAC up-take %
1%
2%
4%
0
15
30
45
60
75
90
Time(min)

*FIG. 3*

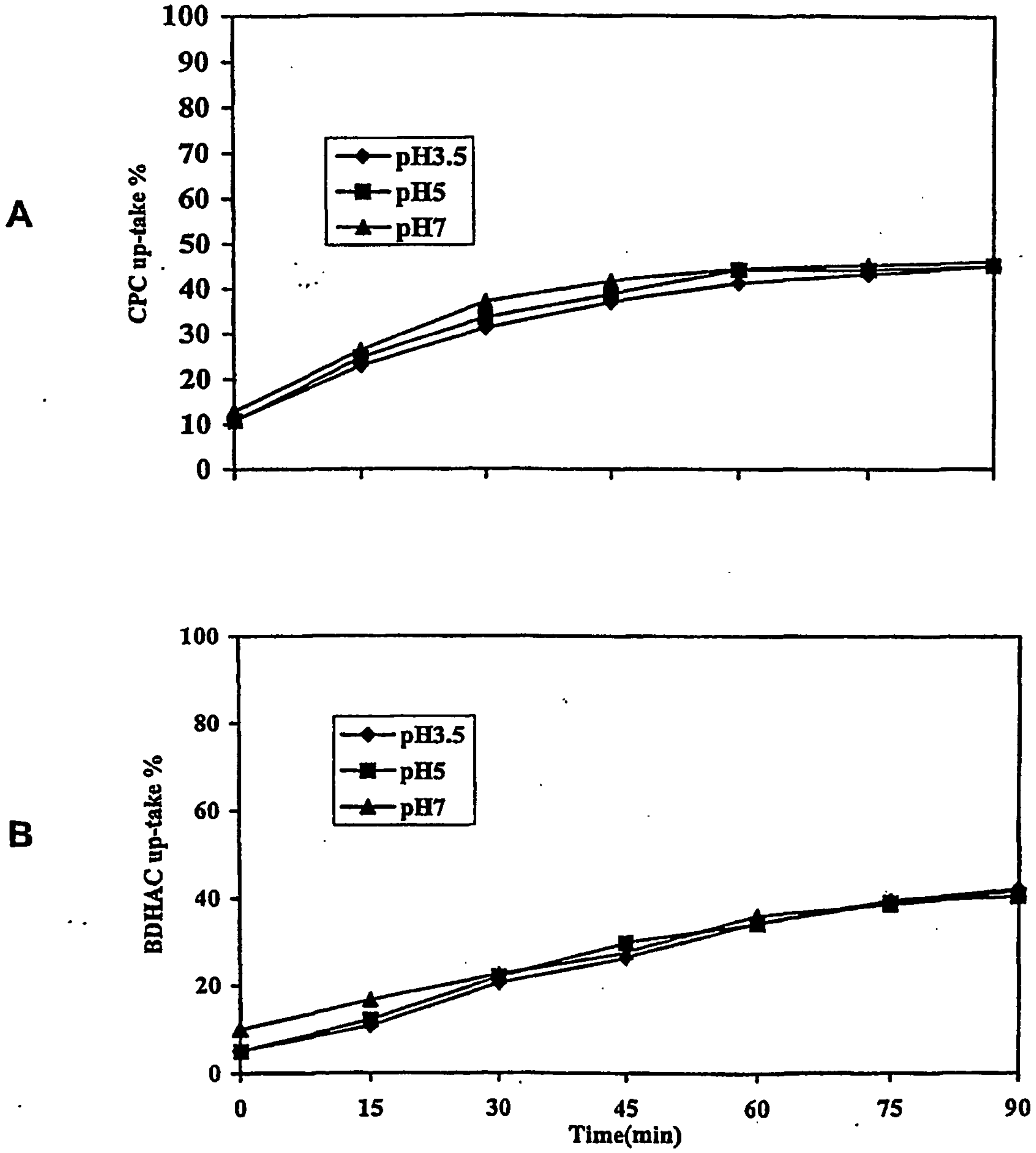
A
CPC up-take %
100
90
80
70
60
50
40
30
20
10
0
pH3.5
pH5
pH7
B
BDHAC up-take %
100
80
60
40
20
0
0
15
30
45
60
75
90
Time(min)

*FIG. 4*

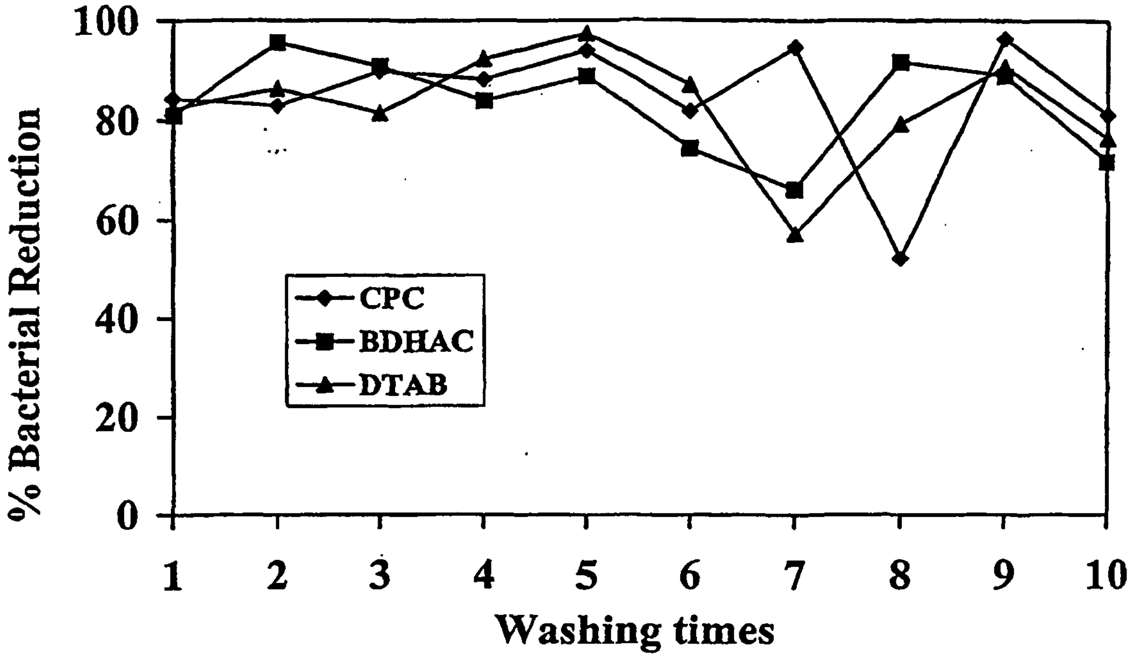
100
80
60
40
20
0
% Bacterial Reduction
CPC
BDHAC
DTAB
1
2
3
4
5
6
7
8
9
10
Washing times

*FIG. 5*

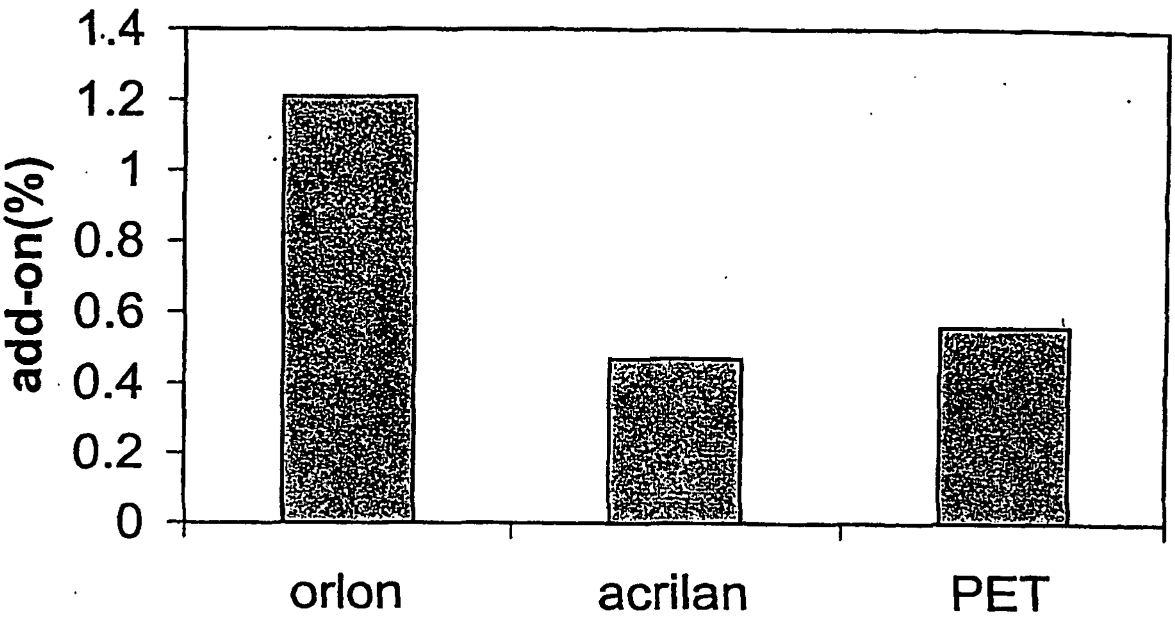


*FIG. 6*

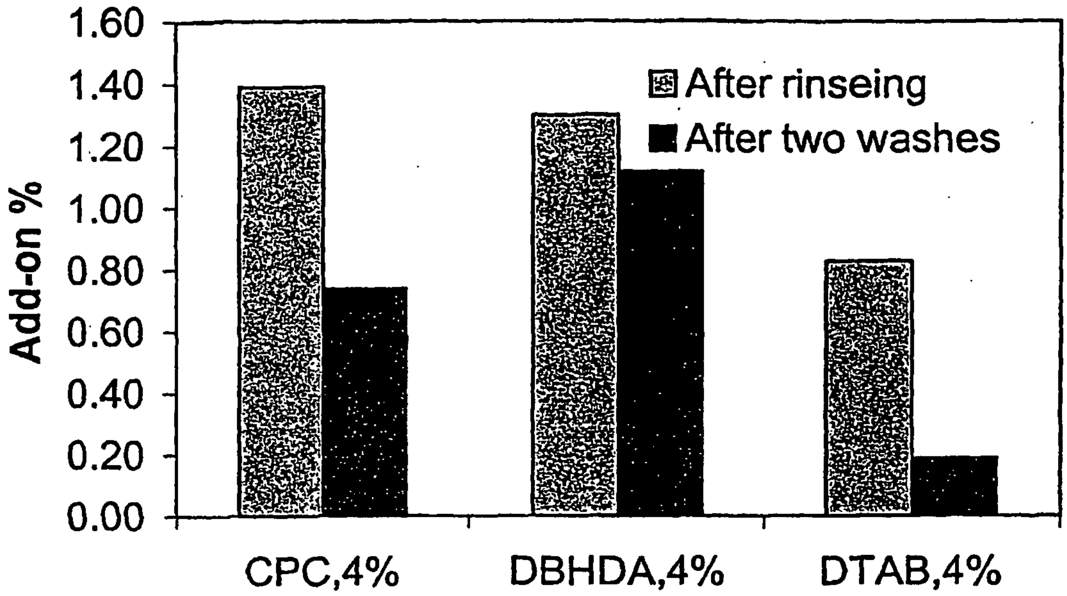
1.60
1.40
1.20
1.00
0.80
0.60
0.40
0.20
0.00
Add-on %
After rinseing
After two washes
CPC,4%
DBHDA,4%
DTAB,4%

*FIG. 7*

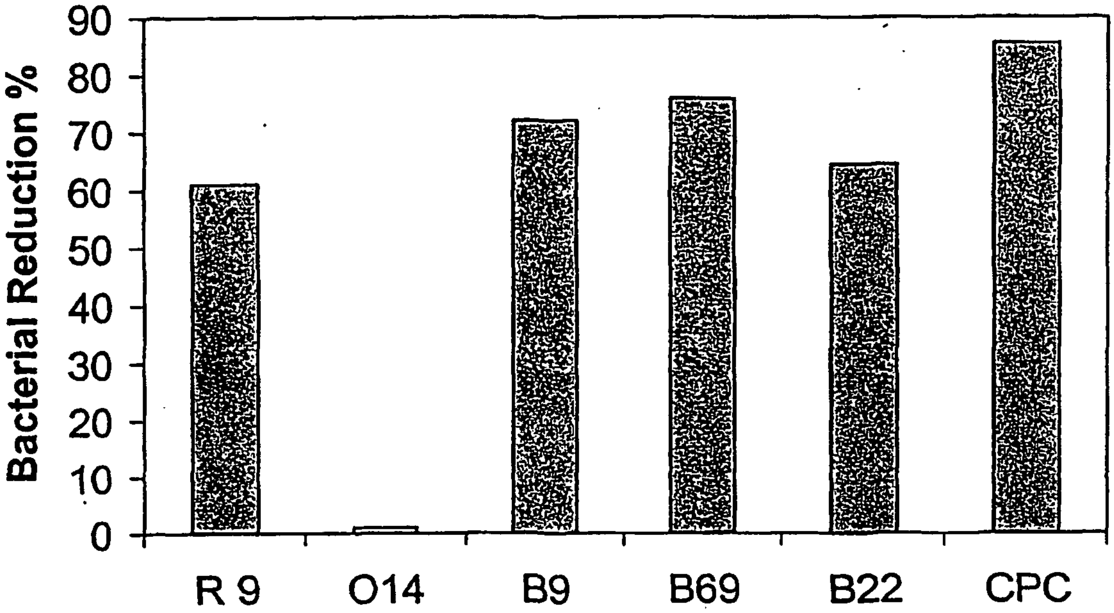
Bacterial Reduction %
90
80
70
60
50
40
30
20
10
0
R 9
O14
B9
B69
B22
CPC

*FIG. 8*

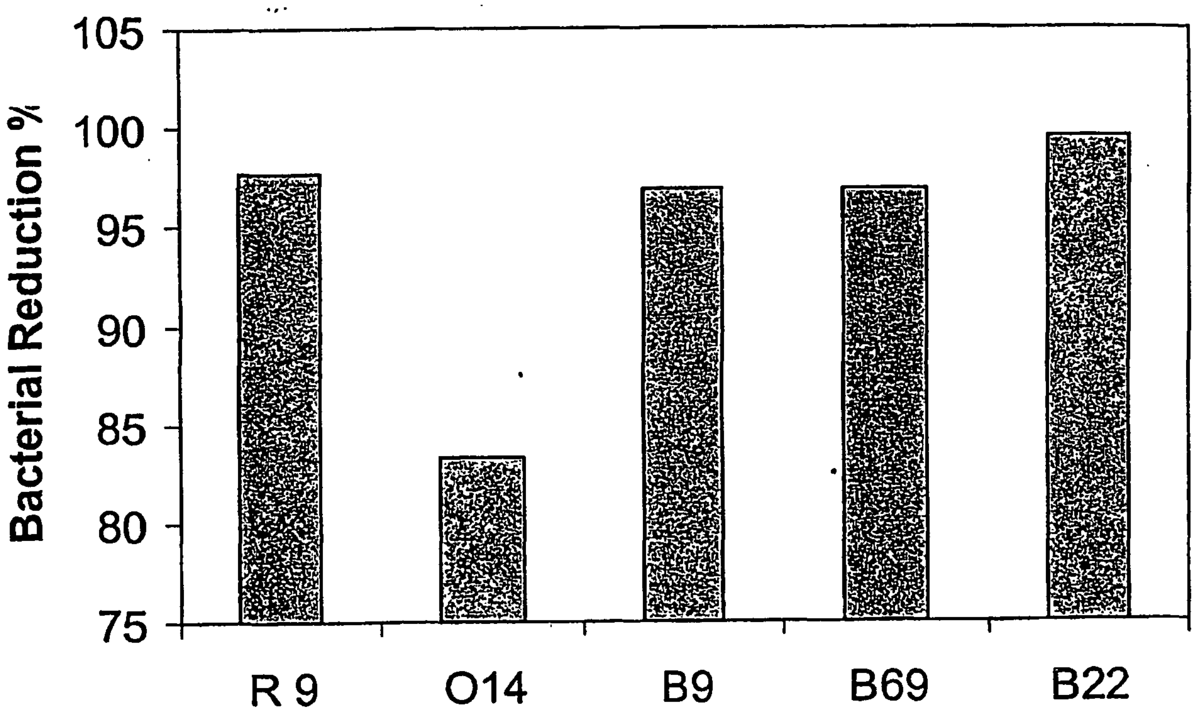


*FIG. 9*